(19) Europäisches Patentamt
European Patent Office
Office européen des brevets

(11) Publication number: **0 107 638**
**B1**

(12) **EUROPEAN PATENT SPECIFICATION**

(45) Date of publication of patent specification: **11.05.88**

(51) Int. Cl.[4]: **B 01 J 23/88,** C 07 C 120/14, C 07 C 45/37

(21) Application number: **83870099.5**

(22) Date of filing: **28.09.83**

(54) **Catalysts for the oxidation and ammoxidation of alcohols.**

(30) Priority: **30.09.82 US 430546**

(43) Date of publication of application:
**02.05.84 Bulletin 84/18**

(45) Publication of the grant of the patent:
**11.05.88 Bulletin 88/19**

(84) Designated Contracting States:
**AT BE CH DE FR GB IT LI NL SE**

(56) References cited:
**EP-A-0 000 564**
**US-A-3 152 997**
**US-A-3 855 153**
**US-A-4 181 629**

(73) Proprietor: **MONSANTO COMPANY**
**Patent Department 800 North Lindbergh Boulevard**
**St. Louis, Missouri 63166 (US)**

(72) Inventor: **Li, Tao Ping**
**293 Heather Crest Drive**
**Chesterfield Missouri 63017 (US)**
Inventor: **Kuechler, Thomas Charles**
**11941 Brookmont Drive**
**Maryland Heights, Missouri 63043 (US)**

(74) Representative: **McLean, Peter et al**
**Monsanto Europe S.A. Patent Department**
**Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 107 638 B1

Courier Press, Leamington Spa, England.

**Description**

Background of the invention

Field of the invention

This invention relates to oxidation and/or ammoxidation catalysts containing the elements iron and molybdenum in a catalytically active oxidized state and to a process for preparing such catalysts. In another aspect, this invention relates to a process for employing such catalysts to effect the oxidation and/or ammoxidation of alcohols. More particularly, this invention relates to a process for employing such catalysts for the ammoxidation of methanol to hydrogen cyanide.

It is known that methanol can be oxidized to the corresponding carbonyl compounds, formaldehyde and formic acid. It is also known that methanol can be ammoxidized to hydrogen cyanide (methanenitrile). The value of such carbonyl compounds and hydrogen cyanide is generally well recognized, with hydrogen cyanide being one of the most valuable compounds available to the chemical industry. Its chief commercial use is as a basic chemical building block for such chemical products as sodium cyanide, potassium cyanide, methyl methacrylate, methionine, triazines, iron cyanides, adiponitrile, and chelates, and other organic compounds, especially acrylonitrile, acetone cyanohydrin, and vinylidene cyanide, the latter compounds being intermediates in the manufacture of certain types of synthetic rubbers, plastics, and fibers.

Hydrogen cyanide is produced primarily by the ammoxidation of methane over platinum metals as catalysts. It has also been produced in substantial quantities as a by-product in the ammoxidation of propylene to acrylonitrile. Recent improvements in the catalysts for the acrylonitrile production, however, have resulted in a significant reduction in the quantity of hydrogen cyanide by-product, while, at the same time, demand has increased. As a result, it is highly desirable to provide more efficient catalysts for the production of hydrogen cyanide from readily available raw materials.

Other nitriles of value which may be produced using the catalysts and process of this invention include acetonitrile from ethanol, acrylonitrile from allyl alcohol, and benzonitrile from benzyl alcohol.

Description of the prior art

Various catalytic processes are known for the oxidation and/or ammoxidation of methanol. Such processes commonly react methanol or a methanol-ammonia mixture with oxygen in the vapor phase in the presence of a catalyst.

Many catalysts are disclosed as suitable in the oxidation and ammoxidation of methanol. In Japanese Kokai patent Sho 51[1976]-10200, a metal oxide catalyst consisting of antimony and at least one additional element from iron, cobalt, nickel, manganese, zinc, and uranium, with the atomic ratio of antimony and the additional elements varying from 1/10 to 10/1, preferably 1/2 to 6/1, is disclosed. The catalyst can be used with or without a support, with silica being a preferred support. A catalyst containing tellurium oxide and molybdenum oxide and optionally at least one of the oxides of tungsten, vanadium, chromium, manganese, iron, cobalt, nickel, zinc, tin, bismuth, and antimony is disclosed as suitable for use in a methanol ammoxidation process to produce hydrogen cyanide in Japanese Kokai patent Sho 51[1976]-99700. In Japanese Kokai patent Sho 53[1978]-149900, a catalyst prepared by spray-drying and calcining a homogeneously mixed solution of silica sol, iron, and molybdenum compounds is disclosed for use in a methanol ammoxidation process. Japanese Kokai patent Sho 54[1979]-126698 discloses a catalyst supported on 30—70 wt.% silica represented by the empirical formula:

$$A_aMoBi_bFe_fNa_nP_pO_q$$

wherein A is at least one element chosen from among potassium, rubidium, cesium, molybdenum, bismuth, iron, sodium, phosphorus, and oxygen, a, b, f, n, p, and q represent the number of atoms of, respectively, A, bismuth, iron, sodium, phosphorus, and oxygen per one (1) atom of molybdenum, with a being a number within the range of 0—0.05, and b, f, and n are numbers that are determined with the respective formulaes of

$$b=\frac{(1-X)(1-Y)(1-Z)}{Y}+0.5Z+p$$

$$f=\frac{X(1-Y)(1-Z)}{Y}$$

$$n=0.5Z$$

In these formulaes, X and Y are numbers inside a square wherein the four coordinate points of (0.35, 0.40), (0.35, 0.65), (0.80, 0.40), and (0.80, 0.65) can be connected, Z is a number within the range of 0—0.6, p is a number within the range of 0—0.2, and q is a number taken to satisfy the valences of the elements in the catalyst.

Although the yield and selectivity of the above-described catalysts are generally satisfactory, the commercial utility of a catalyst system is highly dependent upon the cost of the system, the conversion of the reactant(s), the yield of the desired product(s), and the stability of the catalyst during operation. In many cases, a reduction in the cost of a catalyst system on the order of a few cents per pound or a small percent increase in the yield of the desired product represents a tremendous commercial economical advantage. And since it is well known that the economics of manufacturing processes dictate increasingly higher yields and selectivities in the conversion of reactants to products in order to minimize the difficulties attending the purification of the product(s) and handling of large recycle streams, research efforts are continually being made to define new or improved catalyst systems and methods and processes of making new and old catalyst systems to reduce the cost and/or upgrade the activity and selectivity of such catalyst systems. The discovery of the improved catalyst of the present invention is therefore believed to be a decided advance in the state of the art.

Summary of the invention

It is an object of this invention to provide stabilized catalysts containing the elements iron and molybdenum in a catalytically active oxidized state useful in the preparation of nitriles by ammoxidation of alcohols, characterized by high activity and selectivity to the nitriles.

Another object of this invention is to provide catalysts which are useful for the oxidation of alcohols to the corresponding carbonyl compounds.

Still another object of this invention is to provide catalysts which are useful for the ammoxidation of methanol to hydrogen cyanide.

Yet another object of this invention is to provide catalysts which are useful for the oxidation of methanol to formaldehyde and/or formic acid.

A further object of this invention is to provide an improved process for the preparation of catalysts containing oxygen, iron, and molybdenum.

Yet another object of this invention is to provide an ammoxidation process which employs such catalysts.

To achieve these and other objects which will become apparent from the accompanying description and claims, a catalyst is provided which contains the elements iron and molybdenum in a catalytically active oxidized state represented by the empirical formula:

$$Fe_aMo_bO_c$$

wherein a is 1, b is 1 to 5, and c is a number taken to satisfy the valence requirements of Fe and Mo in the oxidation states in which they exist in the catalysts. According to the present invention, such catalysts are prepared by

(a) mixing an aqueous solution of ammonium molybdate with an aqueous solution of ferric nitrate in amounts sufficient to cause formation of an iron/molybdenum oxide precipitate;

(b) heating the aqueous iron/molybdenum oxide mixture at a temperature and for a time sufficient to convert the initially formed brown precipitate to a tan-to-yellow precipitate;

(c) separating the tan-to-yellow iron/molybdenum oxide precipitate from the aqueous phase of the mixture;

(d) washing occluded impurities from the tan-to-yellow iron/molybdenum oxide precipitate;

(e) forming an aqueous slurry of the tan-to-yellow iron/molybdenum oxide precipitate and water;

(f) forming the aqueous iron/molybdenum oxide slurry into dry particles; and

(g) calcining the dry particles to form the active catalyst.

Description of the preferred embodiments

In accordance with this invention, catalysts containing iron and molybdenum in a catalytically active oxidized state useful for the oxidation and/or ammoxidation of alcohols are represented by the empirical formula:

$$Fe_aMo_bO_c$$

wherein a is 1, b is 1 to 5, and c is a number taken to satisfy the valence requirements of Fe and Mo in the oxidation states in which they exist in the catalysts.

According to the present invention, such catalysts are prepared by

(a) mixing an aqueous solution of ammonium molybdate with an aqueous solution of ferric nitrate in amounts sufficient to cause formation of an iron/molybdenum oxide precipitate;

(b) heating the aqueous iron/molybdenum oxide mixture at a temperature and for a time sufficient to convert the initially formed brown precipitate to a tan-to-yellow precipitate;

(c) separating the tan-to-yellow iron/molybdenum oxide precipitate from the aqueous phase of the mixture;

(d) washing occluded impurities from the tan-to-yellow iron/molybdenum oxide precipitate;

(e) forming an aqueous slurry of the tan-to-yellow iron/molybdenum oxide precipitate and water;

(f) forming the aqueous iron/molybdenum oxide slurry into dry particles; and
(g) calcining the dry particles to form the active catalyst.

The catalysts of the present invention are prepared under narrowly prescribed critical conditions. An aqueous iron/molybdenum oxide slurry is prepared by combining sufficient amounts of aqueous solutions of ammonium molybdate [$(NH_4)_2MoO_4$; prepared by dissolving molybdenum trioxide in aqueous ammonia] and ferric nitrate [$Fe(NO_3)_3 \cdot 9H_2O$]. Since the ammonium molybdate is essentially neutral with respect to pH and the ferric nitrate solution is highly acidic, the resulting aqueous iron/molybdenum oxide slurry or mixture will also be highly acidic, that is to say, it will have a pH less than about 3.

Upon mixing the two aqueous solutions, an initial brown precipitate forms. It has been found to be critical to the performance of the catalysts that this initially formed brown precipitate is converted to a final tan-to-yellow precipitate. This conversion is readily accomplished by heating the mixture at a temperature (usually 95—100°C) and for a time sufficient to effect such conversion (usually 1—2 hours).

The tan-to-yellow iron/molybdenum oxide precipitate is separated from the aqueous phase of the mixture and thoroughly washed to remove occluded impurities, most notably ammonium nitrate. The thoroughly washed precipitate is thereafter slurried in water. If a supported catalyst is desired, the iron/molybdenum oxide precipitate is slurried into an aqueous mixture of a suitable support material. The resultant slurry is ball milled for about 16—18 hours or until the solid particles are reduced to a size less than about 10 microns in diameter.

At this point, the intimately mixed slurry (including support material, if employed) is heated to remove the bulk of the aqueous phase. The concentrated slurry contains a certain amount of water and it is desirable to remove this water by some form of drying process to form a dry catalyst precursor. This can take the form of a simple oven drying process in which the water-containing solid phase is subjected to a temperature that is sufficiently high to vaporize the water and completely dry the solid phase.

An alternate drying process which may be employed is the so-called spray-drying process. In this process, which is preferred for use in the present invention, water-containing solid phase particles are sprayed into contact with a hot gas (usually air) so as to vaporize the water. The drying is controlled by the temperature of the gas and the distance the particles travel in contact with the gas. It is generally desirable to adjust these parameters to avoid too rapid drying as this results in a tendency to form dried skins on the partially dried particles of the solid phase which are subsequently ruptured as water occluded within the particles vaporizes and attempts to escape. At the same time, it is desirable to provide the catalyst in a form having as little occluded water as possible. Therefore, where a fluidized bed reactor is to be used and microspheroidal particles are desired, it is advisable to choose conditions of spray drying with a view toward achieving substantial complete drying without particle rupture.

Following the drying operation, the catalyst precursor is calcined to form the active catalyst. The calcination is usually conducted in air at essentially atmospheric pressure and at a temperature in the range from about 500°C to about 1150°C, preferably from about 750°C to about 900°C. The time to complete the calcination can vary and will depend upon the temperature employed. In general, the time period can be anything up to about 24 hours, but for most purposes, a time period from about 1 hour to about 3 hours at the designated temperatures is sufficient.

The catalyst can be employed without a support and will display excellent activity. However, in some applications, it may be advantageous to include in the catalyst a support material which functions by providing a large surface area for the catalyst and by creating a harder and more durable catalyst for use in the highly abrasive environment of a fluidized bed reactor. This support material can be any of those commonly proposed for such use, such as, for example, silica, zirconia, alumina, titania, antimony pentoxide sol, or other oxide substrates. From the point of view of availability, cost, and performance, silica is usually a satisfactory support material and is preferably in the form of silica sol for easy dispersion.

The proportions in which the components of the supported catalyst are present can vary widely, but it is usually preferred that the support material provides from about 10% to about 90% and more preferably about 35% to about 65% by weight of the total combined weight of the catalyst and the support. To incorporate a support into the catalyst, the support material is mixed with water. The iron/molybdenum oxide precipitate is then slurried with the aqueous support material mixture at a rate sufficient to maintain slurry fluidity and prevent gel formation.

As previously stated, catalysts according to this invention are those represented by the empirical formula:

$$Fe_aMo_bO_c$$

wherein a is 1, b is 1 to 5, and c is a number taken to satisfy the valence requirements of Fe and Mo in the oxidation states in which they exist in the catalyst. In more preferred embodiments of such catalysts, a is 1 and b is 1.5.

The catalyst preparation of this invention yields improved catalysts that exhibit exceptional utility in the production of nitriles from alcohols. Alcohols suitable for use in this invention are those characterized as primary alcohols. Primary alcohols are defined as those alcohols which contain the carbinol group, —$CH_2OH$, that is, those having at least two (2) hydrogen atoms attached to the hydroxy-substituted carbon. Nonlimiting representatives of such alcohols include methanol, ethanol, 1-propanol, 1-butanol, 2-methyl-1-propanol (isobutyl alcohol), 2-propen-1-ol (allyl alcohol), benzyl alcohol, and the like. Of particular importance is the production of hydrogen cyanide (methanenitrile) from methanol and in the discussion which follows, specific reference is made to that process although it should be understood that the described catalysts are also useful for the ammoxidation of other suitable alcohols and for the oxidation of such alcohols to the corresponding carbonyl compound(s), for example, aldehydes and carboxylic acids.

In the most frequently used ammoxidation processes, a mixture of alcohol, ammonia, and oxygen (or air) is fed into a reactor and through a bed of catalyst particles at elevated temperatures. Such temperatures are usually in the range of about 350°C to about 550°C and preferably about 400°C to about 500°C, and the pressure is from about 1 atmosphere to about 6 atmospheres (14.7 psia to about 88.2 psia; 100 kPa to about 600 kPa), preferably about 18.2 psia to about 29 psia (125 kPa to about 200 kPa). The ammonia and alcohol are required stoichiometrically in equimolar amounts, but it is usually necessary to operate with a mol ratio of ammonia to alcohol in excess of 1 to reduce the incidence of side reactions. Likewise, the oxygen and alcohol are required stoichiometrically in equimolar amounts but again, a mol ratio of oxygen to alcohol in excess of 1 is usually employed. The feed mixture is commonly introduced into the catalyst bed at W/F (defined as the weight of the catalyst in grams divided by the flow of reactant stream in ml/sec at standard temperature and pressure) in the range of about 2 g-sec/ml to about 15 g-sec/ml, preferably from about 4 g-sec/ml to about 10 g-sec/ml.

The ammoxidation reaction is exothermic and for convenience in heat distribution and removal, the catalyst bed is desirably fluidized. However, fixed catalyst beds may also be employed with alternative heat removal means such as cooling coils within the bed.

The catalysts as prepared by the improved process of this invention are particularly well adapted for use in such a process in that improved yields of and selectivities to the desired product(s) are experienced due to the unique and novel preparative procedure employed herein.

The following examples illustrating the best presently known methods of practicing this invention are described in order to facilitate a clear understanding of the invention. It should be understood, however, that the expositions of the application of the invention, while indicating preferred embodiments, are given by way of illustration only and are not to be construed as limiting the invention since various changes and modifications within the spirit of the invention will become apparent to those skilled in the art from this description.

As used herein, the following terms are defined in the following manner:

1. "W/F" is defined as the weight of the catalyst in grams divided by the flow rate of the reactant stream in ml/sec measured at STP, the units being g-sec/ml.
2. "Methanol ($CH_3OH$) conversion" is defined as:

$$\frac{\text{mols } CH_3OH \text{ in feed} - \text{mols } CH_3OH \text{ in effluent}}{\text{mols } CH_3OH \text{ in feed}} \times 100$$

3. "Hydrogen cyanide (HCN) selectivity" is defined as:

$$\frac{\text{mols HCN in effluent}}{\text{mols } CH_3OH \text{ converted}} \times 100$$

4. "Hydrogen cyanide (HCN) yield" is defined as:

$$\frac{\text{mols HCN formed}}{\text{mols } CH_3OH \text{ in feed}} \times 100$$

In the following paragraphs, the catalysts (approximately 25 g in each case), were evaluated in a fluidized bed reaction vessel having an inside diameter of about 13 mm to determine nitrile selectivity and yield and alcohol conversion. A reactant mixture of 17.5—18.5 volume % $O_2$, 7—8 volume % methanol ($CH_3OH$), 7—9 volume % ammonia ($NH_3$), and the balance helium was passed upward through the catalyst bed at a rate sufficient to give the value of W/F desired. The temperature was maintained between about 400°C and about 500°C (preferred temperatures) and the pressure at about 125 kPa (18.2 psia) to about 200 kPa (29 psia) unless otherwise noted.

Example 1

A catalyst having the following composition

$$Fe_2(MoO_4)_3\text{—}50\%\ SiO_2$$

was prepared in the following manner.

A solution of 730.0 g (5.069 mols) of molybdenum trioxide ($MoO_3$) dissolved in 736 ml of 28% aqueous ammonia and 1600 ml of water was added to a stirred solution of 1366.0 g (3.381 mols) of ferric nitrate nonahydrate [$Fe(NO_3)_3 \cdot 9H_2O$] dissolved in 2400 ml of water at a rate sufficient to prevent gel formation. A light brown precipitate formed during the addition. The mixture, having a pH of 2.3, was heated at about 100°C until the initially formed light brown precipitate turned bright yellow (approximately 2 hours). The vessel was covered during the heating period to minimize loss of water. The mixture was cooled and suction filtered. The collected iron/molybdenum oxide (ferric molybdate) precipitate was washed with 4000 ml of water.

The ferric molybdate precipitate was slurried into 2500.0 g of 40% aqueous silica sol (Nalcoag 2327), its pH having been adjusted to 2 with nitric acid, at a rate sufficient to maintain the fluidity of the slurry. The slurry was transferred to a ball jar and ball milled for about 16 hours, or until the solid particles were reduced to a size less than 10 microns. The ball milled slurry was transferred to a covered jar, heated for 2 hours, and concentrated to a volume of about 2700 ml. The slurry was then spray dried at a temperature of about 150°C. The dried particles were calcined at 785°C for 2 hours in air to produce the active catalyst supported on 50% by weight silica.

Example 2

To demonstrate the improvement of the catalysts of the present invention, a catalyst was prepared for comparison purposes according to the procedure described in Practical Example 1 of Japanese Kokai Patent Sho 53[1978]-149900.

A solution of 57.5 g (0.0465 mol) ammonium paramolybdate [$(NH_4)_6Mo_7O_{24} \cdot 4H_2O$] dissolved in 750 ml of water was added, with vigorous stirring, to a solution of 394.7 g (0.977 mol) of ferric nitrate nonahydrate [$Fe(NO_3)_3 \cdot 9H_2O$] in 750 ml of water. The solution (no precipitation occurred) had a pH of 1. Silica sol (312.5 g; 40% silica; Nalcoag 2327) was then added. The mixture was concentrated at about 90°C to a volume of about 350 ml and spray dried. The spray dried material was heated at 250°C for 12 hours and calcined at 500°C for 16 hours. The finished catalyst, having an iron/molybdenum atom ratio of 3/1, was supported on 50% by weight silica.

Example 3

To further demonstrate the improvement of the catalysts of the present invention, a catalyst was prepared for comparison purposes according to the procedure described in Practical Example 1 of Japanese Kokai Patent Sho 53[1978]-149900 except that the iron/molybdenum atom ratio was 1/1.5, the stoichiometry of ferric molybdate.

A solution of 103.3 g (0.0836 mol) of ammonium paramolybdate in 750 ml of water was added, with vigorous stirring, to a solution of 157.9 g (0.391 mol) of ferric nitrate nonahydrate dissolved in 300 ml of water. Upon completion of the addition and mixing, the mixture, having a pH of 1, became a greenish gel. The mixture was diluted with water to a total volume of about 1800 ml to increase its fluidity. Silica sol (288.0 g; 40% silica; Nalcoag 2327) was then added and the mixture concentrated to a volume of about 1500 ml. The mixture was spray-dried, heated at 250°C for 12 hours and calcined at 500°C for 16 hours. The active catalyst contained 50% silica by weight.

Example 4

The catalysts from Examples 1—3 were separately charged to reaction vessels described above and used to convert methanol to hydrogen cyanide (HCN). The parameters and results are shown in Table 1.

TABLE 1

| Catalyst | 1 | | 2[1] | | 3[2] | |
|---|---|---|---|---|---|---|
| Fe/Mo atom ratio | 1/1.5 | | 3/1 | | 1/1.5 | |
| Run | 1a | 1b | 2a[3] | 2b[3] | 3a[3] | 3b[3] |
| Reaction temperature, °C | 449 | 445 | 414 | 336 | 450 | 391 |
| Pressure, $10^2$ kPa | 1.70 | 1.70 | 1.70 | 1.29 | 1.70 | 1.70 |
| Feed, vol. % | | | | | | |
| $CH_3OH$ | 7.0 | 7.0 | 7.0 | 7.0 | 7.0 | 7.0 |
| $NH_3$ | 7.1 | 7.1 | 7.1 | 7.1 | 7.1 | 7.1 |
| $O_2$ | 18.0 | 18.0 | 18.0 | 18.0 | 18.0 | 18.0 |
| He | 67.9 | 67.9 | 67.9 | 67.9 | 67.9 | 67.9 |
| W/F, g-sec/ml | 4.5 | 3.3 | 2.5 | 2.5 | 2.5 | 2.5 |
| $CH_3OH$ Conv., mol % | 97.6 | 94.9 | 100.0 | 98.1 | 100.0 | 99.5 |
| HCN | | | | | | |
| Selectivity, mol % | 87.7 | 90.4 | 14.5 | 36.9 | 56.5 | 61.3 |
| Yield, mol % | 85.6 | 85.8 | 14.5 | 36.2 | 56.5 | 61.0 |

[1] Catalyst prepared according to the procedure described in Practical Example 1 of Japanese Kokai Patent Sho 53[1978]-149900.
[2] Catalyst prepared according to the procedure described in Practical Example 1 of Japanese Kokai Patent Sho 53[1978]-149900, except that the Fe/Mo atom ratio was 1/1.5.
[3] Comparative Example.

From the results, it can be seen that the catalyst prepared according to the improved procedure of the present invention (Catalyst 1) gave substantially higher hydrogen cyanide selectivity and yield as compared to catalysts of the prior art (Catalysts 2 and 3), thereby demonstrating the improvement exhibited by the catalysts of the present invention.

Example 5

To show the effectiveness of the catalysts of the present invention to catalyze alcohol oxidation reactions, the catalyst from Example 1 above was charged to the reaction vessel described above and used to convert methanol to formaldehyde (HCHO). The parameters and results are shown in Table 2.

TABLE 2

| | |
|---|---|
| Catalyst | 1 |
| Fe/Mo atom ratio | 1/1.5 |
| Reaction temperature, °C | 429 |
| Pressure, $10^2$ kPa | 1.08 |
| Feed, vol. % | |
| $CH_3OH$ | 7.0 |
| $O_2$ | 18.0 |
| He | 75.0 |
| W/F, g-sec/ml | 4.0 |
| $CH_3OH$ Conv., mol % | 97.2 |
| HCHO | |
| Selectivity, mol % | 95.4 |
| Yield, mol % | 92.7 |

Thus, it is apparent that there has been provided in accordance with the present invention, catalysts and a process for using same that fully satisfy the objects and advantages set forth hereinabove.

**Claims**

1. A catalyst containing iron and molybdenum in a catalytically active oxidized state useful for the oxidation and ammoxidation of alcohols, which catalyst is represented by the empirical formula:

$$Fe_aMo_bO_c$$

wherein a is 1, b is 1 to 5, and c is a number taken to satisfy the valence requirements of Fe and Mo in the oxidation states in which they exist in the catalyst, the catalyst being obtainable by a process comprising:

(a) mixing an aqueous solution of ammonium molybdate with an aqueous solution of ferric nitrate in amounts sufficient to cause formation of an iron/molybdenum oxide precipitate;

(b) heating the aqueous iron/molybdenum oxide mixture at a temperature and for a time sufficient to convert the initially formed brown precipitate to a tan-to-yellow precipitate;

(c) separating the tan-to-yellow iron/molybdenum oxide precipitate from the aqueous phase of the mixture;

(d) washing occluded impurities from the tan-to-yellow iron/molybdenum oxide precipitate;

(e) forming an aqueous slurry of the tan-to-yellow iron/molybdenum oxide precipitate and water;

(f) forming the aqueous iron/molybdenum oxide slurry into dry particles; and

(g) calcining the dry particles to form the active catalyst.

2. A catalyst of Claim 1 wherein a is 1 and b is 1.5.

3. A catalyst of either Claim 1 or Claim 2, obtainable by said process, wherein the aqueous ammonium molybdate solution is prepared by dissolving molybdenum trioxide in aqueous ammonia.

4. A catalyst of any of Claims 1 to 3 that contains a support material comprising from 10% to 90% by weight of the total weight of the catalyst.

5. A catalyst of Claim 4 wherein the support material comprises from 35% to 65% by weight of the total weight of the catalyst.

6. A catalyst of either Claim 4 or Claim 5 wherein the support material is silica.

7. A catalyst of any of Claims 4 to 6, obtainable by the said process, wherein the support material is incorporated into the catalyst by adding the iron/molybdenum oxide precipitate to an aqueous support material mixture during the formation of the aqueous slurry of the tan-to-yellow iron/molybdenum oxide precipitate.

8. A catalyst of any of the preceding claims, obtainable by the said process wherein the dry particles are formed by spray drying the aqueous slurry.

9. A catalyst of any of the preceding claims, obtainable by the said process wherein the dry particles are calcined at a temperature from 500°C to 1150°C.

10. A catalyst of Claim 9 wherein the calcination temperature is from 750°C to 900°C.

11. A process for preparing a catalyst containing iron and molybdenum in a catalytically active oxidized state represented by the empirical formula:

$$Fe_aMo_bO_c$$

wherein a is 1, b is 1 to 5, and c is a number taken to satisfy the valence requirements of Fe and Mo in the oxidation states in which they exist in the catalyst, the process being as defined in Claim 1, Claim 3 or any of Claims 7 to 10.

12. A process for the production of a nitrile which comprises reacting at an elevated temperature in the vapor phase an alcohol, ammonia, and molecular oxygen in the presence of a catalyst according to any of Claims 1 to 10.

13. A process of Claim 12 wherein the alcohol is methanol and the nitrile is hydrogen cyanide.

14. A process of either Claim 12 or Claim 13 that is conducted at a pressure of 100 kPa to 600 kPa and a temperature of 350°C to 550°C.

15. A process of Claim 14 wherein the pressure is from 125 kPa to 200 kPa and the temperature is from 400°C to 500°C.

16. A process of any of Claims 12 to 15 wherein the flow rate W/F is in the range of 2 g-sec/ml to 15 g-sec/ml.

17. A process for the production of a carbonyl compound which comprises reacting at an elevated temperature in the vapor phase an alcohol and molecular oxygen in the presence of a catalyst according to any of Claims 1 to 10.

18. A process of Claim 17 wherein the alcohol is methanol and the carbonyl compound is formaldehyde.

19. A process of either Claim 17 or Claim 18 wherein the process is conducted at a pressure of 100 kPa to 600 kPa and a temperature of 350°C to 550°C.

20. The process of any of Claims 17 to 19 wherein the flow rate W/F is in the range of 2 g-sec/ml to 15 g-sec/ml.

**Patentansprüche**

1. Katalysator enthaltend Eisen und Molybdän in katalytisch aktivem oxidierten Zustand, der für die Oxidation und Ammoxidation von Alkoholen verwendbar ist, welcher Katalysator die empirische Formel

$$Fe_aMo_bO_c$$

aufweist, worin a 1 ist, b 1 bis 5 ist und c eine Zahl ist, die gewählt wurde, um den Wertigkeitsanforderungen von Fe und Mo in den Oxidationszuständen, in denen sie im Katalysator vorhanden sind, zu entsprechen, welcher Katalysator nach einem Verfahren erhältlich ist, das das

(a) Mischen einer wässerigen Ammoniummolybdatlösung mit einer wässerigen Ferrinitratlösung in Anteilen, die ausreichend sind, die Bildung eines Eisen/Molybdänoxidniederschlages zu bewirken,

(b) Erhitzen der wässerigen Eisen/Molybdänoxidmischung auf eine Temperatur und während eines Zeitraumes, die ausreichend sind, den zuerst gebildeten braunen Niederschlag in einen gelbbraun-bis-gelben Niederschlag umzuwandeln,

(c) Abtrennen des gelbbraun-bis-gelben Eisen/Molybdänoxidniederschlages von der wässerigen Phase der Mischung,

(d) Auswaschen von vom gelbbraun-bis-gelben Eisen/Molybdänoxidniederschlag eingeschlossenen Verunreinigungen,

(e) Bilden einer wässerigen Aufschlämmung aus dem gelbbraun-bis-gelben Eisen/Molybdänoxidniederschlag und Wasser,

(f) Bilden von trockenen Teilchen der wässerigen Eisen/Molybdänoxidaufschlämmung und

(g) Kalzinieren der trockenen Teilchen unter Bildung des aktiven Katalysators umfaßt.

2. Katalysator nach Anspruch 1, worin a 1 ist und b 1,5 ist.

3. Katalysator nach Anspruch 1 oder 2, erhältlich nach dem genannten Verfahren, wobei die wässerige Ammoniummolybdatlösung durch Lösen von Molybdäntrioxid in wässerigem Ammoniak hergestellt wird.

4. Katalysator nach einem der Ansprüche 1 bis 3, der ein Trägermaterial enthält, das 10 bis 90 Gew.% des Gesamtgewichtes des Katalysators ausmacht.

5. Katalysator nach Anspruch 4, worin das Trägermaterial 35 bis 65 Gew.% des Gesamtgewichtes des Katalysators ausmacht.

6. Katalysator nach Anpsruch 4 oder 5, worin das Trägermaterial Kieselerde ist.

7. Katalysator nach einem der Ansprüche 4 bis 6, erhältlich nach dem gennanten Verfahren, wobei das Trägermaterial dem Katalysator durch Zusetzen des Eisen/Molybdänoxidniederschlages zu einer wässerigen Trägermaterialmischung während der Bildung der wässerigen Aufschlämmung des gelbbraun-bis-gelben Eisen/Molybdänoxidniederschlages einverleibt wird.

8. Katalysator nach einem der vorhergehenden Ansprüche, erhältlich nach dem genannten Verfahren, wobei die trockenen Teilchen durch Sprühtrocknen der wässerigen Aufschlämmung gebildet werden.

9. Katalysator nach einem der vorhergehenden Ansprüche, erhältlich nach dem genannten Verfahren, wobei die trockenen Teilchen bei einer Temperatur von 500 bis 1150°C kalziniert werden.

10. Katalysator nach Anspruch 9, worin die Kalzinierungstemperatur 750 bis 900°C beträgt.

11. Verfahren zum Herstellen eines Katalysators enthaltend Eisen und Molybdän in katalytisch aktivem oxidierten Zustand, dargestellt durch die empirische Formel

$$Fe_aMo_bO_c$$

aufweist, worin a 1 ist, b 1 bis 5 ist und c eine Zahl ist, die gewählt wurde, um den Wertigkeitsanforderungen von Fe und Mo in den Oxidationszuständen, in denen sie im Katalysator vorhanden sind, zu entsprechen, wobei das Verfahren wie in Anspruch 1, Anspruch 3 oder einem der Ansprüche 7 bis 10 definiert ist.

12. Verfahren zur Herstellung eines Nitrils, welches das Umsetzen eines Alkohols, von Ammoniak und molekularem Sauerstoff in Anwesenheit eines Katalysators nach einem der Ansprüche 1 bis 10 bei erhöhter Temperatur in der Dampfphase umfaßt.

13. Verfahren nach Anspruch 12, worin der Alkohol Methanol ist und das Nitril Cyanwasserstoff ist.

14. Verfahren nach Anspruch 12 oder 13, das bei einem Druck von 100 bis 600 kPa und bei einer Temperatur von 350 bis 550°C durchgeführt wird.

15. Verfahren nach Anspruch 14, worin der Druck 125 bis 200 kPa ist und die Temperatur 400 bis 500°C beträgt.

16. Verfahren nach einem der Ansprüche 12 bis 15, worin die Strömungsrate W/F im Bereich von 2 bis 15 g-s/ml liegt.

17. Verfahren zur Herstellung einer Carbonylverbindung, welches das Umsetzen eines Alkohols und von molekularem Sauerstoff in Anwesenheit eines Katalysators nach einem der Ansprüche 1 bis 10 bei erhöhter Temperatur in der Dampfphase umfaßt.

18. Verfahren nach Anspruch 17, worin der Alkohol Methanol ist und die Carbonylverbindung Formaldehyd ist.

19. Verfahren nach Anspruch 17 oder 18, wobei das Verfahren bei einem Druck von 100 bis 600 kPa und bei einer Temperatur von 350 bis 550°C durchgeführt wird.

20. Verfahren nach einem der Ansprüche 17 bis 19, worin die Strömungsrate W/F im Bereich von 2 bis 15 g-s/ml liegt.

**Revendications**

1. Catalyseur contenant du fer et du molybdène dans un état oxydé catalytiquement actif utilisable pour l'oxydation et l'ammoxydation d'alcools, lequel catalyseur est représenté par la formule brute:

$$Fe_aMo_bO_c$$

dans laquelle a est 1, b est 1 à 5, et c est un nombre choisi pour satisfaire aux exigences de valence de Fe et Mo dans les états d'oxydation dans lesquels ils existent dans le catalyseur, le catalyseur pouvant être obtenu par un procédé comprenant:

(a) le mélange d'une solution aqueuse de molybdate d'ammonium avec une solution aqueuse de nitrate ferrique dans des quantités suffisantes pour provoquer la formation d'un précipité d'oxyde de fer/molybdène;

(b) le chauffage du mélange aqueux d'oxyde de fer/molybdène à une température et pendant un temps suffisants pour transformer le précipité brun initialement formé en un précipité jaune-brun à jaune;

(c) la séparation du précipité d'oxyde de fer/molybdène jaune-brun à jaune de la phase aqueuse du mélange;

(d) l'élimination par lavage des impuretés occluses du précipité d'oxyde de fer/molybdène jaune-brun à jaune;

(e) la formation d'une bouillie aqueuse du précipité d'oxyde de fer/molybdène jaune-brun à jaune et d'eau;

(f) la transformation de la bouillie aqueuse d'oxyde de fer/molybdène en particules sèches; et

(g) la calcination des particules sèches pour former le catalyseur actif.

2. Catalyseur selon la revendication 1, dans lequel a est 1 et b est 1,5.

3. Catalyseur selon l'une quelconque des revendications 1 ou 2, pouvant être obtenu par ce procédé, dans lequel la solution aqueuse de molybdate d'ammonium est préparée en dissolvant du trioxyde de molybdène dans de l'ammoniaque.

4. Catalyseur selon l'une quelconque des revendications 1 à 3, qui contient une matière de support constituant de 10% à 90% en poids du poids total du catalyseur.

5. Catalyseur selon la revendication 4, dans lequel la matière de support constitue de 35% à 65% en poids du poids total du catalyseur.

6. Catalyseur selon l'une quelconque des revendications 4 ou 5, dans lequel la matière de support est de la silice.

7. Catalyseur selon l'une quelconque des revendications 4 à 6, pouvant être obtenu par ce procédé, dans lequel la matière de support est incorporée au catalyseur en ajoutant un précipité d'oxyde fer/molybdène à un mélange aqueux de matière de support au cours de la formation de la bouillie aqueuse du précipité d'oxyde de fer/molybdène jaune-brun à jaune.

8. Catalyseur selon l'une quelconque des revendications précédentes, pouvant être obtenu par ce procédé, dans lequel des particules sèches sont formées en séchant par pulvérisation la bouillie aqueuse.

9. Catalyseur selon l'une quelconque des revendications précédentes, pouvant être obtenu par ce procédé, dans lequel les particules sèches sont calcinées à une température de 500°C à 1150°C.

10. Catalyseur selon la revendication 9, dans lequel la température de calcination est de 750°C à 900°C.

11. Procédé pour préparer un catalyseur contenant du fer et du molybdène dans un état oxydé catalytiquement actif représenté par la formule brute:

$$Fe_aMo_bO_c$$

dans laquelle a est 1, b est 1 à 5, et c est un nombre choisi pour satisfaire aux exigences de valence de Fe et Mo dans les états d'oxydation dans lesquels ils existent dans le catalyseur, le procédé étant tel que défini dans la revendication 1, la revendication 3 ou l'une quelconque des revendications 7 à 10.

12. Procédé pour la préparation d'un nitrile, qui consiste à faire réagir à une température élevée, en phase vapeur, un alcool, de l'ammoniac, et de l'oxygène moléculaire en présence d'un catalyseur suivant l'une quelconque des revendications 1 à 10.

13. Procédé selon la revendication 12, dans lequel l'alcool est le méthanol et le nitrile est l'acide cyanhydrique.

14. Procédé selon l'une quelconque des revendications 12 ou 13, qui est effectué sous une pression de 100 kPa à 600 kPa et à une température de 350°C à 550°C.

15. Procédé selon la revendication 14, dans lequel la pression est de 125 kPa à 200 kPa et la température est de 400°C à 500°C.

16. Procédé selon l'une quelconque des revendications 12 à 15, dans lequel le débit P/D est dans l'intervalle de 2 g-sec/ml à 15 g-sec/ml.

17. Procédé pour la préparation d'un composé carbonylé, qui consiste à faire réagir à température élevée, en phase vapeur, un alcool et de l'oxygène moléculaire en présence d'un catalyseur selon l'une quelconque des revendications 1 à 10.

18. Procédé selon la revendication 17, dans lequel l'alcool est le méthanol et le composé carbonylé est le formaldéhyde.

19. Procédé selon l'une quelconque des revendications 17 ou 18, dans lequel le procédé est effectué sous une pression de 100 kPa à 600 kPa et à une température de 350°C à 550°C.

20. Procédé selon l'une quelconque des revendications 17 à 19, dans lequel le débit P/D est dans l'intervalle de 2 g-sec/ml à 15 g-sec/ml.